Europäisches Patentamt

European Patent Office

Office européen des brevets

(11) Publication number: **0 107 498**
**B1**

# (12) EUROPEAN PATENT SPECIFICATION

(45) Date of publication of patent specification: **16.05.90**

(51) Int. Cl.⁵: **A 61 K 37/66**

(21) Application number: **83306443.9**

(22) Date of filing: **24.10.83**

(54) Synergistic human interferon activity.

(30) Priority: **25.10.82 US 436758**

(43) Date of publication of application:
**02.05.84 Bulletin 84/18**

(45) Publication of the grant of the patent:
**16.05.90 Bulletin 90/20**

(84) Designated Contracting States:
**AT BE CH DE FR GB IT LI LU NL SE**

(56) References cited:
**EP-A-0 087 686**

**"Human Lymphokines", Acad. Press 1982, pp. 539-551**

**Recent Results Cancer Res. 1980, 75, 267-212**

**J. Exp. Med. 159, 828-843 (1984)**

**Cancer Res. 42, 869-875. 1982**

**"Interferon", Karger, Basel, S. 52-62**

(73) Proprietor: **GENENTECH, INC.**
**460 Point San Bruno Boulevard**
**South San Francisco California 94080 (US)**

(72) Inventor: **Czarniecki, Christine W.**
**15 San Antonio Place**
**San Francisco California 94133 (US)**
Inventor: **Rinderknecht, Ernst H.**
**812 Bauer Drive**
**San Carlos California 94070 (US)**

(74) Representative: **Goldin, Douglas Michael et al**
**J.A. KEMP & CO. 14, South Square Gray's Inn**
**London WC1R 5EU (GB)**

(56) References cited:
**BIOLOGICAL ABSTRACTS, Vol. 69, No. 7, 1980, page 4534, abstract no. 42391, PHILADELPHIA, (US), W.R. FLEISCHMANN et al.:Potentiation of interferon activity by mixed preparations of fibroplast and immune interferon. & Infect Immun 26(1): 248-253, 1979**

Courier Press, Leamington Spa, England.

# EP 0 107 498 B1

(56) References cited:

CHEMICAL ABSTRACTS, Vol. 98, No. 3, January 17, 1983, page 384, abstract no. 15122p, COLUMBUS, OHIO (US), L.A. SCHWARZ et al.: "Potentiation of interferon action" & Tex. Rep. Biol. Med. 1982, 41, 298-306

THE JOURNAL OF IMMUNOLOGY, Vol. 129, No. 1, July 1982, pages 76-80, BALTIMORE, MARYLAND (US), G.R. KLIMPEL et al.: "Gamma interferon (IFNgamma) and IFNalpha/bêta suppress murine myeloid colony formation (CFU-C): Magnitude of suppression is dependent upon level of colony-stimulating factor (CSF)

INFECTION AND IMMUNITY, Vol. 40, No. 1, April 1983, pages 35-38, D.A. WEIGENT et al.: "Potentation of lymphocyte natural killing by mixtures of alpha or beta interferon with recombinant gamma interferon".

## Description

The present invention is based upon the discovery that a combination of human gamma interferon (IFN-γ), sometimes referred to as human immune interferon, with human beta interferon (IFN-ß) or a hybrid thereof results in biological activities surprisingly much higher than could have been fairly predicted based upon their respective activities when tested alone. The results hereof demonstrate a true synergistic effect from the interferon proteins themselves, because very pure preparations, containing for all intents and purposes only the respective inteferons, hitherto unobtainable but for the application of recombinant DNA technology, were employed in the testing procedures.

The publications and other materials hereof used to illuminate the background of the invention, and in particular instances, to provide additional details concerning its practice are, for convenience, numerically referenced parenthetically in the following text and respectively grouped in the appended bibliography.

Klimpel et al., J. Immunol., 129, (1982) and Fleischmann, Chem. Abs, 98, 15122 (1983) refer to experiments on animal interferons obtained by extraction from cells which normally produce interferons and tests using these animal interferons on animals cells.

Human interferons have been grouped into three classes based upon differences in observed biological properties and molecular structures. For example, the human alpha interferons comprise a family of proteins which have been shown to possess both antiviral and cell growth antihibitory effects (1—10). Similarly, IFN-β, which has only about 25—30 percent homology generally with IFN-αs, has demonstrated biological activity (11). IFN-γ, until recently the most elusive of the interferons, shares virtually no homology with interferons of the other series, yet also demonstrates activities not unlike in kind those displayed by the other two series of interferons (12).

The IFN-β interferons are produced by, and have been isolated from, cells exposed to viruses. IFN-γ interferon is induced in lymphocytes in response to specific mitogenic stimuli. Interferon preparations obtained from natural sources have been used in studies comparing biological activities (13—16). The results of these studies, although based on mammalian (mouse) protein systems, are not translationally suggestive of counterpart studies which could be conducted with human interferon preparations. Further, these studies were necessarily conducted with relatively impure preparations, further confounding any interpretations of the results as attributable to the interferons qua interferons or to other potantially interfering impurities (proteinaceous or otherwise, for example various lymphokines) or to combination effects of the mixture. (See 17). Furthermore, the cells from which these relatively impure preparations were obtained are known to also produce other substances that exhibit cytopathic effects which would further complicate the situation.

Previous to the production of human interferons via recombinant DNA technology (See 1, 11, 12, 18), definitive determination of the biological properties of natural human interferons has been confounded by the difficulties inherent in obtaining purified materials. And although the enormous potency of interferons has permitted biological analyses even of such impure preparations, it remains uncertain to what extent the results are due wholly or in part to the presence of the impurities (See 19, 20). Protein interactions and mixed activities are possible, if not well known, phenomena. Thus, unequivocal bioactivity results for such (relatively impure) preparations should not be anticipated. Indeed, attempts to arrive at further purified preparations have resulted in activity levels different from those observed with more impure preparations (19).

From studies designed to test the potentiating effect of mixed preparations of interferons (14; 15; mouse) (17; human), again, done with relatively impure materials, conclusions beyond speculation as to cause and effect would not be scientifically grounded. And although these studies provide a bsis for speculation of such potentiation, an actual, positive demonstration has not been shown (14).

The present invention addresses the problems inherent with the studies based on impure materials and provides requisite proof of demonstrated synergistic effect of the interferons qua interferons. With the advent of recombinant DNA technology, it has become possible to produce and isolate human interferons from geneticall altered host cultures in copious quantities and in purities heretofore unobtainable (1, 11, 12, 18). Consequently, it has become possible to test a hypothesis as to the true effect the various human interferons may have on one another. The results of this test are the aspects of the compass of the present invention.

The present invention is directed as a primary aspect, to a composition comprising a pharmaceutically acceptable diluent or carrier and a mixture of pharmaceutically pure human gamma interferon and pharmaceutically pure human beta interferon or a hybrid thereof in a weight ratio of from 2 to 45 parts of gamma interferon to from 0.07 to 0.008 parts of beta interferon or hybrid thereof. The interferons being products of genetically altered host cell culture.

The present invention is being based upon the signal recovery of non-additive, synergistic protentiation of biological activity of IFN-γ in combination with IFN-β interferons or hybrids thereof.

Further the present invention is directed to the preparation of pharmaceutical compositions comprising the interferon combinations described above.

An effective graphic analysis of the responses of two agents hereof in combination involves construction of isobolograms which utilize the data for the effects of each agent separately as well as in combination (see 21—24). This method has been utilized to analyze, for example, the antiproliferative effects of IFN-$\beta_1$ and IFN-$\gamma$. The human IFNs used in one study described herein, IFN-$\beta_1$ and IFN-$\gamma$, were synthesized in E. coli (1, 11, 12, 18) and the interferons were thus for the first time made available for use in greater than 95 percent purity as determined by SDS polyacrylamide gel electrophoresis and HPLC analysis (see 7). IFN comparisons were made on the basis of antiviral units assayed on HeLa cells challenged with vesicular stomatitis virus standardizing IFN-$\gamma$ against the NIH human leukocyte standard (G023-901-527) and IFN-$\beta_1$ against the NIH human fibroblast standard (G023-902-527). The reference standards are available without restriction from the Antiviral Substance Program of the National Institute of Allergy and Infectious Diseases, National Institutes of Health, Washington, D.C. The human melanoma cell line, Hs294T, was initiated and cloned at the Cell Culture Department, Naval Biosciences Laboratory, Oakland, CA and is similarly available from the Biological Carcinogenesis Branch of National Cancer Institute of the National Institutes of Health, Bethesda, Maryland. The origin, characteristics and cytogenetics of this line have been described previously (26, 27). The growth of these cells was shown to be inhibited by partially purified human leukocyte IFN, and this inhibition was not associated with decreased cell viability (27).

The term "genetically altered host cell" refers to microorganisms or other cell cultures including mammalian cultures, containing DNA sequences operably disposed so as to direct the production therein of polypeptides which are not native products of their endogenous processes (heterologous polypeptides). Current refinements in recombinant DNA technology permit the manipulation of DNA sequences in order to cause, for example E. coli, to produce mammalian proteins. Such production, brought about by genetic alteration of the host cell, further defines the term in question in terms of effect.

Recombinant DNA technology also admits the production of heterologous polypeptides in substantially pure form inasmuch as no other proteins of similar origin are produced by the host cell and separation of product from other host cell material is enhanced. Recombinant DNA techniques currently are capable of producing heterologous proteins in purities at least greater than about 95 percent limits defining herein the term "substantially free of impurities". Such limits correspond to activity levels which are orders of magnitude greater than those of interferon preparations obtained from natural source and which do not contain other interfering components such as lymphokines and cytopathic agents. For example, specific activities of recombinant human fibroblast (IFN-$\beta$) interferons have been calculated at about $1 \times 10^8$ Units/mg of total protein; that of human gamma interferon (IFN-$\gamma$) is in the order of at least $10^{-6}$ Units/mg.

One method by which recombinant IFNs useful herein can be purified from e.g., bacteria, is illustrated by the following general scheme, applicable specifically to IFN-$\gamma$:

1. Extraction of the cells in high conductivity lysis buffer (at about pH 8) by passage through a homogenizer at high pressure, cooling the effluent in an ice bath.

2. Precipitation of DNA by polyethylene-imine addition under stirring, for example, at 4°C.

3. pH precipitation of bacterial proteins, again leaving IFN-$\gamma$ in solution.

4. Separation of the solids by centrifugation at 4°C.

5. Concentration of the supernatant (after readjusting the pH) as by ultrafiltration.

6. Dialysis of the concentrate against a low conductivity buffer.

7. Removing solids by centrifugation leaving the IFN-$\gamma$ in solution.

8. Ion exchange chromatography on carboxymethyl cellulose, eluting with a gradient of increasing ionic strength.

9. Chromatography on calcium phosphate gel by eluting with a gradient of increasing ionic strength.

10. Ion exchange chromatography on carboxymethyl cellulose under weak denaturing conditions by eluting with a gradient of increasing ionic strength.

11. Separation by gel filtration chromatography.

The above process enables yields of material of >95 percent purity.

The term "pharmaceutically pure" refers to those interferon preparations hereof defined above, made available by recombinant DNA technology, suitable for unequivocal biological testing as well as for appropriate administration to effect treatment, e.g., in a human being.

Synergistic mixture is defined in terms of a standard, accepted rationale according to which desired, observed effect results from a combination of one-fourth or less of the MIC (minimal inhibitory concentration) of each drug above. (See 24) When the interferons are so tested via a variety of proportions, isobolograms are constructed (see discussion infra.), synergism being demonstrable by a concave curve reaching at least the 0.25 FIC (fractional inhibitory concentration) point. Reference to this standard thus enables the determination of the MICs for the combinations tested, providing the MIC of each component needed to achieve a synergistic mixture.

Based upon the bioassays conducted as described herein on the Hs294T cell line and considering the specific activities above noted, it can be deduced that effective synergistic amounts of about 2 to about 45 micrograms/ml of IFN-$\gamma$ with from about 0.07 to about 0.008 micrograms/ml of IFN-$\beta$. It will be appreciated that actual dosages could vary over a relatively wide range due to the limitations inherent in the above calculations. However, based upon the present disclosure, it would be well within the available skill in the art to determine more precisely those amounts of active components which would enable the synergistic effect of this invention.

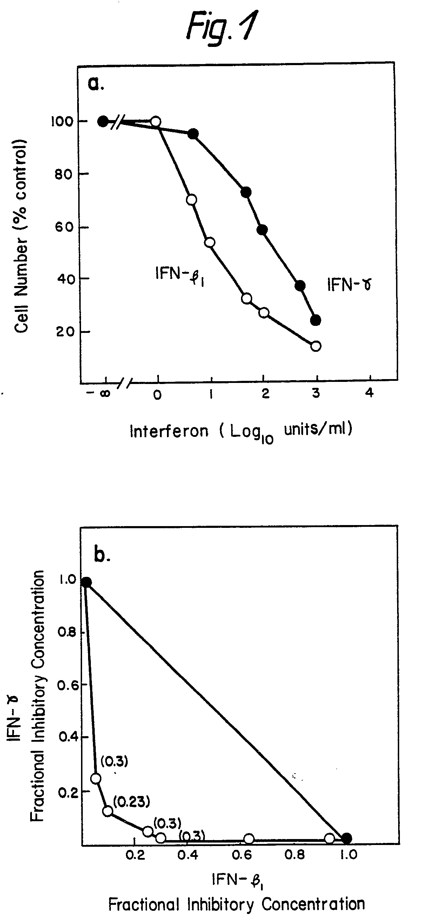

Figure 1 demonstrates the effect of IFN-γ and IFN-β₁ on cell growth. Human melanoma cells (Hs294T) were grown as monolayers in Dulbecco's modified Eagle's medium (Gibco) supplemented with 10 percent fetal calf serum. (a) Dose-response curves. At 24h after cells were seeded ($1 \times 10^5$ cells/35 mm well), culture fluids were replaced with fresh media containing increasing concentrations of either IFN-γ, (●—●) or OFN-β₁ alone (●—●). At 96h after IFN addition, viable cells were counted and are expressed as percent of control cells (receiving no IFN treatment). (b) Isobologram drawn from the 50 percent cell growth inhibitory concentrations obtained from dose response curves of: (i) IFN-γ alone and IFN-β₁ alone (●): (ii) Combinations of increasing concentrations of IFN-β₁ with fixed amounts of IFN-γ (10, 25 or 50 units per ml) (o); and (iii) Combinations of increasing concentrations of IFN-γ with fixed amounts of IFN-β₁ (5, 10, or 15 units per ml (o). Values are expressed in terms of Fractional inhibitory concentrations (FIC). For IFN-γ, FIC of 1.0 represents 200 units per ml; for IFN-β₁, FIC of 1.0 represents 16 units per ml. Values in parentheses represent the FIC index for each combination, as described in the text.

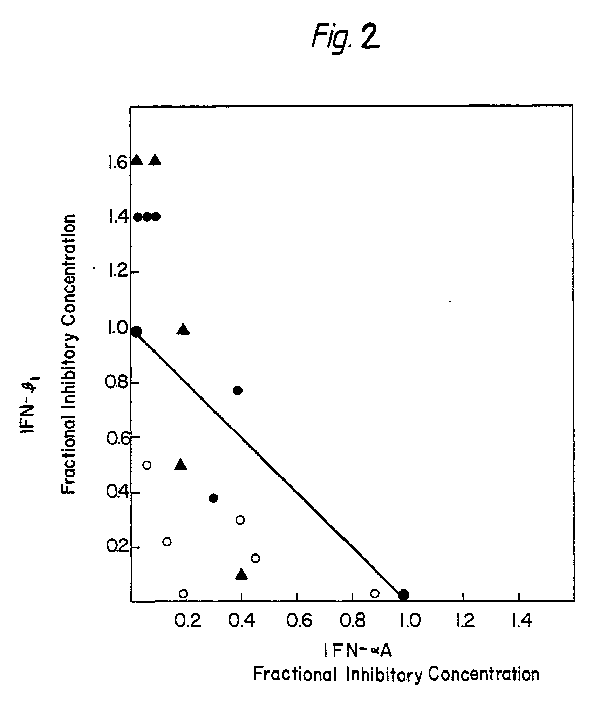

Figure 2 is an isobologram for the effect of IFN-αA and IFN-β₁ on cell growth. The experimental design is described in the legend to Figure 1. Values have been normalized to fractional inhibitory concentrations and represent results from representative experiments: Experiment 1 (●); Experiment 2 (●); Experiment 3 (▲). The concentrations of IFN-αA alone or IFN-β₁ alone resulting in 50 percent inhibition of cell growth varied from 800 to 2,000 units per ml and 10 to 32 units per ml, respectively. These values have been normalized to FIC of 1.0 (●).

The analysis of the interactions of IFN-γ and IFN-β₁ is shown in Figure 1. Hs294T cells were incubated with increasing concentrations of IFN-γ alone, IFN-β₁ alone, or combinations of both IFNs. Combinations consisted of increasing concentrations of IFN-β₁ mixed with fixed subinhibitory doses of IFN-γ and also increasing concentrations of IFN γ mixed with subinhibitor doses of IFN-β₁. The number of viable cells was determined 96h after the addition of IFN and expressed as percentage of cell controls (receiving no IFN treatment) which had gone through 2—3 doublings. The concentrations of the IFNs required to achieve 50 percent inhibition of cell growth are shown in the respective isobolograms. The concentration of each IFN alone needed to achieve 50 percent inhibition of cell growth varied with each experiment, reflecting changes in cell sensitivity to IFN-induced cell growth inhibition during cell passage, as well as possible variation in IFN assays. Therefore, to achieve internal consistency in this type of study, it was necessary to obtain all points used in one isobologram from the same experiment, i.e., replicate cultures were treated with each IFN alone and combinations of IFNs simultaneously to insure that the concentrations of each IFN used in the combinations were all subinhibitory.

The effects of IFN-γ and IFN-β₁ are shown in Figure 1. Cell growth was inhibited by 50 percent when cells were treated with 200 units per ml of IFN-γ or 16 units per ml of IFN-β₁ (Figure 1a). The isobologram drawn from the 50 percent inhibitory points of IFN-γ alone, IFN-β₁ alone and IFN-γ and IFN-β₁ in combination is shown in Figure 1 (b). The points on the abscissa and ordinate represent the amounts of IFN-β₁ alone and IFN-γ alone, respectively, required for 50 percent inhibition of cell growth and have been normalized to fractional inhibitory concentration (FIC) values of 1.0. Any points obtained from combination treatments falling on the line drawn between these points would reprsent an additive effect. Points forming a concave curve to the left of the line would indicate a supra-additive or synergistic response while points forming a convex curve to the right of this line would indicate a sub-additive response implying inhibition or antagonism. As shown in Figure 1(b), the 50 percent points obtained using combinations of IFN-β₁ and IFN-γ formed a concave curve to the left of the additivity line fulfilling the criteria for a synergistic response.

The interactions of IFN-αA and IFN-β₁ in combination in this cell system were also examined. As in Figure 1 cells were treated with various concentrations of IFN-αA and IFN-β₁ alone and in combination. Results obtained from such experiments proved to be inconsistent as shown in Figure 2, with some combinations fitting the definition for synergy and others appearing to be antagonistic. This was a marked contrast to the results from combination treatments of IFN-γ together with IFN-β₁ which reproducibly yielded the synergistic responses shown in Figure 1. The data shown in Figure 2 suggest that the interactions of IFN-αA together with IFN-β₁ are more complex than that of IFN-γ together with IFN-αA or IFN-β₁. The fact that IFN-β₁ can compete for the binding of IFN-αA to cell surface receptors may play a role in this complex response.

In order to differentiate between additivity and synergism, one can utilize the fractional inhibitory concentration (FIC) index which is equal to the sum of the FIC values for the individual drugs used in each combination. A synergistic response is defined by an FIC index of less than 0.5, i.e. when 50 percent inhibition results from a combination of one-fourth or less of the FIC of each drug. An FIC index of 1 defines an additive response. The FIC indices shown in parenthesis for the points in the isobolograms in Figures 1(b) are less than 0.5 thereby fitting the definition of synergistic responses. For example, as shown in Figure 1(b), 50 percent inhibition of cell growth was achieved with a combination dose of 4 units per ml of IFN-β₁ with 10 units per ml of IFN-γ (FIC index of 0.3. Examining the experimental data in this manner provides a means for determining the degree of potentiation between the IFN classes. Thus, FIC indices of approximately 0.3 indicate the degrees of synergy between IFN-γ and IFN-β₁ in this cell system.

The results demonstrate that highly purified preparations of human recombinant IFN-β₁ and IFN-γ can each inhibit the replication of human melanoma cells and that 50 percent inhibition of cell growth was achieved with lower concentration of IFN-β₁ than IFN-γ.

By specific embodiment, it has been demonstrated that human melanoma cells treated with high purity preparations of IFN-γ in combination with IFN-β₁ result in 50 percent inhibition of cell growth, achieved at concentrations much lower than that necessary for each IFN alone. Inasmuch as both antiproliferative and antiviral activities are examples of intrinsic properties of a single IFN molecule (28—31), it follows that treatments with combinations of the different purified IFN types result in similar potentiation of their antiviral activities.

Administration of the components hereof can be via any of the accepted modes of administration for agents which exhibit such activity. These methods include oral, parenteral or topical administrations and otherwise systemic forms. Local or intravenous injection is preferred.

Depending on the intended mode of administration, the compositions used may be in the form of solid, semi-solid or liquid dosage forms, such as, for example, tablets, pills, capsules, powders, liquids and suspensions, preferably in unit dosage forms suitable for single administration of precise dosages. The compositions will include a conventional pharmaceutical carrier or excipient and, in addition, may include other medicinal agents, pharmaceutical agents, carriers and adjuvants. Such excipients may include other proteins, such as, for example, human serum albumin or plasma preparations.

The amount of active compound administration will, of course, be dependent on the subject being treated, the severity of the affliction, the manner of administration and the judgement of the prescribing physician.

For solid compositions, conventional non-toxic solid carriers include for example, pharmaceutical grades or mannitol, lactose starch, or magnesium stearate. Liquid pharmaceutically administerable compositions can, for example, be prepared by dissolving or dispersing, in a carrier, such as, for example, water, saline, aqueous dextrose, glycerol and ethanol to therebyform a solution or suspension. If desired, the pharmaceutical composition to be administered may also contain minor amounts of nontoxic auxiliary substances such as wetting or emulsifying agents and pH buffering agents, for example, sodium acetate or sorbitan monolaurate. Actual methods of preparing such dosage forms are known, or will be apparent, to those skilled in this art; for example, see *Remingtons' Pharmaceutical Sciences*, Mack Publishing Company, Easton, Pennsylvania, 15th Edition, 1975. The composition or formulation to be administered will, in any event, contain a quantity of the active component(s) in an amount effective to achieve the desired effect in the subject being treated.

Bibliography
1. Goeddel *et al., Nature 287,* 411 (1980).
2. Yelverton *et al., Nucleic Acids Research 9,* 731 (1981).
3. Weck *et al., Nucleic Acids Research 9,* 6153 (1981).
4. Weck *et al., J. Gen. Virol, 57,* 233 (1981).
5. Lee *et al., Cancer Research 42,* 1312 (1982).
6. Weck *et al., Infect. Immun. 35,* 660 (1982).
7. Wetzel *et al., J. Interferon Research 1,* 381 (1981).
8. Weck *et al., Injection and Immunity,* in press.
9. Smolin *et al., Arch. Opthalmol. 100,* 481 (1982).
10. Masucci, *et al., Science 209,* 1431 (1980).
11. Goeddel *et al., Nucleic Acids Research 8,* 4057 (1980).
12. Gray *et al., Nature 295,* 503 (1982).
13. Crane *et al., J. Nat. Canc. Inst. 61,* 871 (1978).
14. Fleischmann *et al., Inject. and Immunity 26,* 248 (1979).
15. Fleischmann, *Cancer Research 42,* 869 (1982).
16. Ankel *et al., Proc. Natl. Acad. Sci. (USA) 77,* 2528 (1980).
17. Fleischmann, "Interferon Potentiation: Antiviral and Antitumor Studies," *Interferon Properties, Mode of Action, Production, Clinical Application,* Eds. Munk and Kirchner, S. Karger, Basel, p. 53 et seq.
18. Goeddel *et al., Nature 290,* 20 (1981).
19. Blalock *et al., Cellular Immunology 49,* 390 (1980).
20. deLey *et al., Eur. J. Immun. 10,* 877 (1980).
21. Steel, G. G. *et al. Int. J. Radiation Oncology Biol. Phys. 5,* 85 (1979).
22. Redpath, J. L. *Int. J. Radiation Biol., 38,* 355 (1980).
23. Wilson, G. S. *et al., Principles of Bacteriology, Virology and Immunity* (Topley and Wilson, ed) Vol. I Williams and Wilkens, Baltimore, Med., (1975).
24. Goodman, A., *et al., The Pharmagological Basis of Therapeutics* MacMillan Publication Co., Inc., N.Y., (1980).
25. European Patent Application Publication No. 0051873 (19 May 1982).
26. Creasey, A. A. *et al., In Vitro 15,* 342 (1979).
27. Creasey, A. A. *et al., Proc. Nat. Acad. Sci. (USA) 77,* 1471 (1980).
28. Gresser, I. *et al., Proc. Nat. Acad. Sci. (USA) 66,* 1052 (1970).
29. Stewart II *et al., Nature 262,* 300 (1976).
30. Berg, K. *et al., J. Gen. Virol. 50,* 441 (1980).
31. Evinger, M. *et al., Arch. Biochem. and Biophys. 210,* 319 (1981).

**Claims for the Contracting States: BE, CH, DE, FR, GB, IT, LI, LU, NI, SE**

1. A composition comprising a pharmaceutically acceptable diluent or carrier and a mixture of pharmaceutically pure human gamma interferon and pharmaceutically pure human beta interferon or a hybrid thereof in a weight ratio of from 2 to 45 parts of gamma interferon to from 0.07 to 0.008 parts of beta interferon or hybrid thereof, the interferons being products of genetically altered host cell culture.

2. A composition according to Claim 1, for use in a therapeutic treatment of the human or animal body.

3. Use of a composition according to Claim 1 or Claim 2 in the manufacture of a medicament for use in therapeutic treatment of the human or animal body.

4. A process for producing a composition according to Claim 1 comprising combining the human gamma interferon and the human beta interferon or hybrid thereof and a pharmaceutically acceptable diluent or carrier.

**Claims for the Contracting State: AT**

1. A process for producing a composition comprising a pharmaceutically acceptable diluent or carrier and a mixture of pharmaceutically pure human gamma interferon and pharmaceutically pure human beta interferon or a hybrid thereof in a weight ratio of from 2 to 45 parts of gamma interferon to from 0.07 to 0.008 parts of beta interferon or hybrid thereof, the interferons being products of genetically altered host cell culture comprising combining the human gamma interferon and the human beta interferon or hybrid thereof and a pharmaceutically acceptable diluent or carrier.

2. A process according to Claim 1 for producing a composition for use in a therapeutic treatment of the human or animal body.

3. Use of a composition according to Claim 1 or Claim 2 in the manufacture of a medicament for use in therapeutic treatment of the human or animal body.

**Patentansprüche für die Vertragsstaaten: BE, CH, DE, FR, GB, IT, LI, LU, NL, SE**

1. Präparat, dadurch gekennzeichnet, daß es einen pharmazeutisch annehmbaren Diluenten oder Träger und ein Gemisch aus pharmazeutisch reinem Human-γ-Interferon und pharmazeutisch reinem Human-β-Interferon oder einem Hybrid davon in einem Gewichtsverhältnis von 2 bis 45 Teilen γ-Interferon zu 0,07 bis 0,008 Teilen β-Interferon oder eines Hybrids davon enthält, wobei die Interferone Produkte einer genetisch veränderten Wirtszellkultur sind.

2 Präparat nach Anspruch 1, zur Verwendung bei der therapeutischen Behandlung des menschlichen oder tierischen Körpers.

3. Verwendung eines Präparats nach Anspruch 1 oder 2 zur Herstellung eines Medikaments zur Verwendung bei der therapeutischen Behandlung des menschlichen oder tierischen Körpers.

4. Verfahren zur Herstellung eines Präparats nach Anspruch 1, dadurch gekennzeichnet, daß man das Human-γ-Interferon und das Human-β-Interferon oder ein Hybrid davon und einen pharmazeutisch annehmbaren Diluenten oder Träger vereinigt.

**Patentansprüche für den Vertragsstaat: AT**

1. Verfahren zur Herstellung eines Präparats aus einem pharmazeutisch annehmbaren Diluenten oder Träger und einem Gemisch aus pharmazeutisch reinem Human-γ-Interferon und pharmazeutisch reinem Human-β-Interferon oder einem Hybrid davon in einem Gewichtsverhältnis von 2 bis 45 Teilen γ-Interferon zu 0,07 bis 0,008 Teilen β-Interferon oder eines Hybrids davon, wobei die Interferone Produkte einer genetisch veränderten Wirtszellkultur sind, dadurch gekennzeichnet, daß man das Human-γ-Interferon und das Human-β-Interferon oder ein Hybrid davon und einen pharmazeutisch annehmbaren Diluenten oder Träger vereinigt.

2. Verfahren nach Anspruch 1 zur Herstellung eines Präparats zur Verwendung bei der therapeutischen Behandlung des menschlichen oder tierischen Körpers.

3. Verwendung eines Präparats nach Anspruch 1 oder 2 zur Herstellung eines Medikaments zur Verwendung bei der therapeutischen Behandlung des menschlichen oder tierischen Körpers.

**Revendications pour les Etats Contractants: BE, CH, DE, FR, GB, IT, LI, LU, NL, SE**

1. Composition comprenant un diluant ou support pharmaceutiquement acceptable et un mélange de gamma-interféron human pharmaceutiquement pur et de bêta-interféron humain pharmaceutiquement pur ou d'un de ses hybrides, en un rapport pondéral de 2 à 45 parties de gamma-interféron à 0,07—0,008 partie de bêta-interféron ou d'un de ses hybrides, les interférons étant des produits d'une culture de cellules-hôtes modifiées génétiquement.

2. Composition suivant la revendication 1, destinée à être utilisée dans un traitement thérapeutique de l'organisme d'un être humain ou d'un animal.

3. Utilisation d'une composition suivant la revendication 1 ou la revendication 2, dans la production d'un médicament destiné à être utilisé dans un traitement thérapeutiquement de l'organisme d'un être humain ou d'un animal.

4. Procédé de production d'une composition suivant la revendication 1, consistant à mélanger le gamma-inteféron humain et le bêta-interféron humain ou un de ses hybrides et un diluant ou support pharmaceutiquement acceptable.

**Revendications pour l'Etat Contractant: AT**

1. Procédé de production d'une composition comprenant un diluant ou support pharmaceutiquement acceptable et un mélange de gamma-interféron humain pharmaceutiquement pur et de béta-interféron humain pharmaceutiquement pur, ou d'un de ses hybrides, en un rapport pondéral de 2 à 45 parties de gamma-interféron à 0,07—0,008 partie de béta-interféron ou d'un de ses hybrides, les interférons étant des produits d'une culture de cellules-hôtes modifiées génétiquement, consistant à mélanger le gamma-interféron human et le béta-interféron human ou un de ses hybrides et un diluant ou support pharmaceutiquement acceptable.

2. Procédé suivant la revendication 1, pour la production d'une composition destinée à être utilisée dans un traitement thérapeutiquement de l'organisme d'un être humain ou d'un animal.

3. Utilisation d'une composition suivant la revendication 1 ou la revendication 2, dans la production d'un médicament destiné à être utilisé dans un traitement thérapeutique de l'organisme d'un être humain ou d'un animal.

Fig.1

## Fig. 2